# EUROPEAN PATENT SPECIFICATION

(11) **EP 0 542 246 B1**
(45) Date of publication and mention of the grant of the patent: **17.09.1997**
(21) Application number: 92119323.1
(22) Date of filing: 12.11.1992
(51) Int. Cl.: A61M 25/00

(54) **Vascular access catheter**
Vaskularer Zugangskatheter
Cathéter d'accès vasculaire

(30) Priority: 13.11.1991 US 791871
(43) Date of publication of application: 19.05.1993
(73) Proprietor: Becton Dickinson and Company, Franklin Lakes, New Jersey 07417-1880 (US)
(72) Inventor: Pearsall, Harold I., Xenia, Greene County, Ohio 45385 (US)
(74) Representative: Bosotti, Luciano

(56) References cited:
- EP-A- 0 303 487
- EP-A- 0 334 640
- EP-A- 0 363 953
- US-A- 3 866 599

## Description

### BACKGROUND AND DESCRIPTION OF THE INVENTION

This invention relates to a catheter for introduction into a patient, and more particularly to catheters formed with a very soft tip configured in a manner which reduces to a very great extent the trauma to the patient during insertion and placement of the catheter in the patient body.

The force applied for advancing a catheter into the blood vessel of a patient is transmitted by the catheter body and, is exerted against the vessel wall into which the catheter tip is to be inserted. Thus, the catheter tip, when in contact with a blood vessel wall, transmits the force applied to the catheter itself. This force can be amplified, if the catheter tip has sharp concentrated corners.

Other ways of exerting pressure against a vessel wall during the placement procedure by a catheter is periodic flexing of the vessel wall against the catheter tip which results from the heart beating or patient's movement during the actual time when the catheter is placed in the patient. This constant irritation is detrimental to the patient's well being and it is to these particular problems that the present invention is directed.

There are many prior art arrangements for providing a reduction in trauma to a patient during the insertion and continued placement of a catheter in the patient. One such arrangement is taught in U.S. Patent No. 5,049,138 which issued September 17, 1991. This particular arrangement includes a dissolvable tip. That is, the tip is made of a material which is soluble in body fluids. Thus, when it becomes wet, it becomes slippery for ease of insertion into the body of a patient and then the tip dissolves.

There are certain limitations to such a procedure in that the dissolved tip reduces control of the catheter during the placement procedure. Moreover, the material comprising the tip may be somewhat toxic to the patient.

Another recent patent is U.S. Patent 5,045,072 which issued September 3, 1991 and which includes a flexible front end or distal tip for a catheter. This particular tip is directed particularly to a formulation having a percent of radiopaque material to be used as a positioning arrangement for the placement procedure. The tip is comprised of a material which is substantially rigid in the range of 75A-85A durometer for proper control of the tip during the insertion and placement.

A further patent is U.S. Patent 5,017,259 which issued May 21, 1991, and which teaches a catheter with a tip member comprised of a relatively soft material. The tip is joined to the catheter body using an adhesive. The tip is comprised of a softer material but includes a sharp front end cut-off of the distal tip. Because of this, the patient is subjected to the sharp front corners of the flat distal end of the soft tip during the insertion procedure. Moreover, there is a definite transition abutment at the margin between the two parts.

More specifically, the present invention relates to a vascular access catheter having the features set forth in the preamble of Claim 1, which is known, e.g. from EP-A-0 303 487.

While the above patent documents teach inventions which have the effect of reducing substantially the trauma to patients during the insertion and placement procedure of catheters, they still do not provide the very soft distal end in combination with proper control during the placement procedure. In other words, either the soft tip is of a substantial length for providing a reduced trauma procedure which reduces proper control during placement, or the material provides proper control but the material is relatively hard and/or rigid for the insertion thus increasing trauma. Moreover, all of the tips discussed have cut-off front ends which present sharp edges to the patient during the insertion and placement procedure.

### DETAILED DESCRIPTION OF THE INVENTION

With this invention, having the further features set forth in Claim 1, by contrast, a vascular access catheter is provided, having a tip comprised of an extremely soft Shore A durometer material which is preferably of a specific length. This controlled length provides proper control during the insertion procedure, but reduces, nevertheless, trauma because of its very soft nature. Moreover, the front end of the tip is configured to have rounded edges for presentation to the patient's skin and blood vessels during the insertion and placement procedure.

As a further feature of the invention, the catheter body itself is of a very soft material relatively speaking as related to the tip itself and the two parts are preferably joined together by RF bonding, as discussed above, which reduces to a minimum the amount of the body of the tip and the body of the catheter that are exposed to melting during the joining of the two parts together. This has the effect of reducing any distortion of the surfaces of the two parts at the joint so that there is, again, a reduction in irritation to the patient along this surface during the insertion and placement procedures.
More particularly, this invention relates to soft tip catheters in which the tip is formed of a very soft Shore A material within a specific range and the tip is of a specific length dimension in order to provide proper softness as required for introduction into the patient, while at the same time providing proper control of the distal end of the catheter during the placement procedure.

The arrangement herein includes a specific method for joining the catheter body and the tip body together by RF bonding. This allows for a very small exposure of the length of the catheter tip and the catheter body to any energy requirement for the bonding procedure. Because of this, very little disfigurement takes place of the outer surface of the tip and the adjoining catheter body so that there is, again, a substantial reduction in any irritation to the patient during the insertion and placement procedure.

In considering generally the configuration for the catheter of the invention, one may note that the catheter body is comprised of a soft polyurethane material within the range of between about 80-90 Shore A. This limits the amount of force transmitted, because it will bend during manipulation of the catheter during use. Second, an even softer polyurethane material is utilized for the soft tip. This is less than 80 Shore A.

As a further feature of the invention, and as discussed above, the length of the soft tip is within the range of about 0.635 and 1.27 cm (about 0.25 and 0.50 inches). Because of this specific length, it has been found that the catheter tip exerts a minimum pressure to the blood vessel wall of the patient while at the same time it does not kink or collapse and prevent functioning of the catheter. Longer lengths than this cause collapse or kinking upon occasion, and thus reduce the functioning of the catheter.

Again, the tip of the catheter is rounded to eliminate the sharp cut-off edges ordinarily provided on the distal end of catheters. It has been found that the rounded front end together with the controlled length of the tip and its very soft Shore A durometer polyurethane material has the effect of providing proper control without traumatizing the patient and providing minimum risk of vessel wall perforation or damage.

The tip comprising of a very soft polyurethane material is preferably bonded to the polyurethane catheter body using an RF bonding technique. The RF bonding technique enables the application of a controlled concentrated heat to melt very narrow bands of both the catheter body and the soft tip materials at the junction point. Molten catheter body and soft tip materials at the junction point blend at a molecular level forming an interpenetrating macromolecular structure, thus achieving a superior bonding.

Another advantage of the use of RF bonding is that different amounts of heat may be applied to body and tip materials to accommodate their varying melt temperatures, given that each one is comprised of a different Shore A durometer and may have different additives for specific reasons.

In considering generally the conditions for carrying out the bonding procedure, there may be a warm-up period of, for example 190-220 milliamps at 38 units. This is followed by a drop to 40-60 milliamps for the actual bonding at 55 units. Thereafter, there is a cool down period of 99 units. It will be understood by practitioners-in-the-art that these levels are representative only, and that variations will take place depending on such factors as the Shore A durometer of the polymer material involved, as well as wall thickness, and additive variations in the polymer.

### DESCRIPTION OF THE DRAWINGS

Fig. 1 is a somewhat diagrammatic schematic longitudinal illustration of the distal end of the catheter of the invention.
Fig. 1a shows, in detail, the configuration of the curved structure of the front end of the tip of the invention;
Fig. 2 is a schematic side elevational view of the joining procedure for joining the tip of the catheter of the invention to the distal end of the body of the catheter of the invention; and
Fig. 3 is a diagrammatic illustration of the distal end of the catheter of the invention with the body and the tip joined together, and illustrating schematically a method of joining the two parts together.

### DETAILED DESCRIPTION OF THE INVENTION

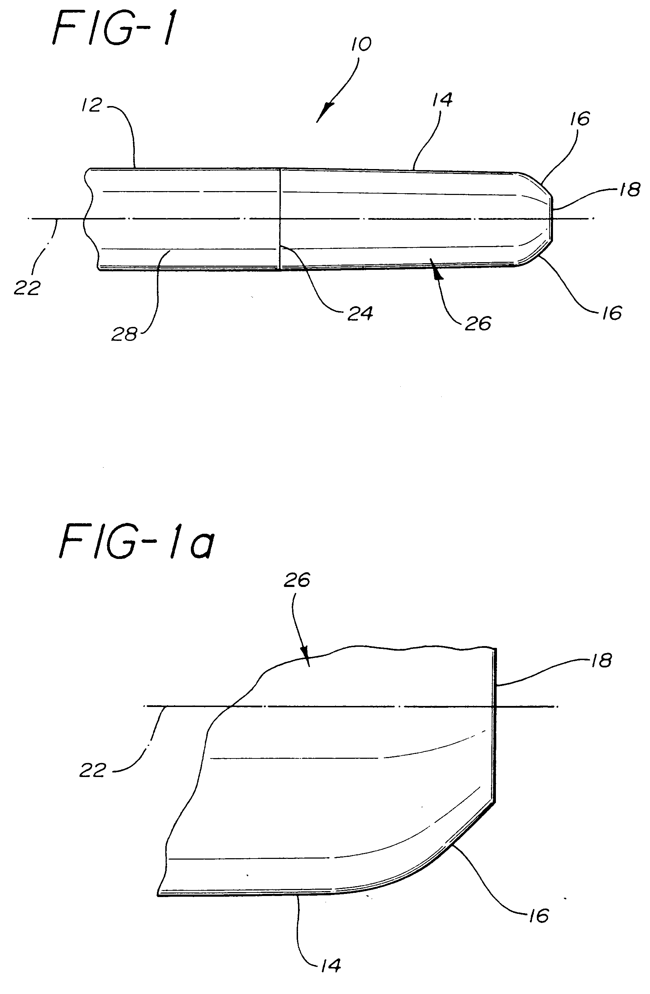

Referring to the drawings in which like reference characters refer to like parts throughout the several views thereof, Fig. 1 shows the front or distal end of the catheter of the invention generally designated at 10, with the front or distal end of the catheter body 28 being joined at 24 to a catheter tip 26. As can be seen in Fig. 1, in this side elevational view of the front or distal end of the catheter of the invention, the surface 12 of the front end of catheter body 28 is relatively horizontal, while the outer surface 14 of the catheter tip 26 gradually tapers to the front end 18 of the tip 26.

As can be seen in Fig. 1, the front end edge of tip 26 is rounded at 16 from the tapered surface 14 of the tip 26 to the front end 18 thereof. This gradual curve is emphasized more clearly in the enlarged view shown in Fig. 1a. Axis 22 of the catheter body-tip distal end shown in Figs. 1 and 1a is merely placed to illustrate more clearly, the configuration of the catheter of the invention. Rounded front end surface 16 is annular, circumscribing axis 22.

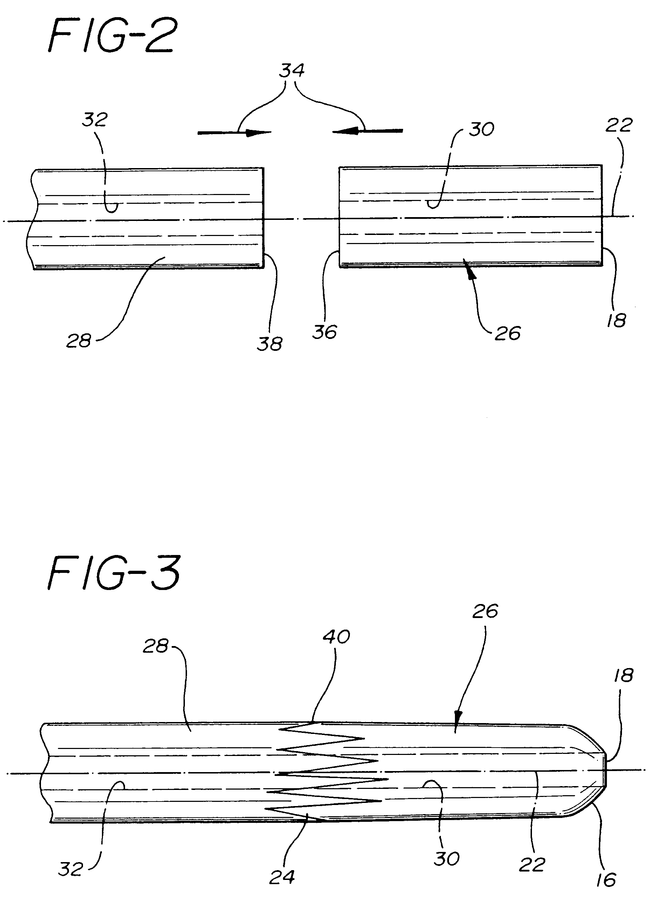

Referring now to Fig. 2, a procedure is illustrated diagrammatically or schematically for the joining of the body 28 and the tip 26 together. That is, the opposed surfaces 36, 38 of the tip 26 and body 28 respectively are joined together by being moved toward each other as illustrated by the arrows 34. Once the two surfaces 36, 38 are joined, they are exposed to RF energy to cause the area immediately adjacent the two surfaces 36, 38 to melt to join the two parts together.

Because RF bonding is used, a very small portion of the length of the tip 26 and the body 28 are exposed to the energy related to the RF bonding. Because of this, surface 40, as shown in Fig. 3, has very little disfiguration which would cause any kind of irritation to the patient during the insertion and placement procedure. As can be seen in Figs. 2 and 3, the body 28 and tip 26 have lumens 32, 30 respectively, which are also joined together during the joining procedure, so that there is a continuous flow through the catheter once the joining has taken place and once the catheter has been inserted and placed properly for use in the patient.

Once the body 28 and the tip 26 are joined at their respective surfaces 36, 38 then, preferably, tip distal end 18 is inserted into a mold to form the rounded front end surface 16. Alternatively, the rounded front end surface 16 may be formed prior to joining body 28 and tip 26 together.

In either case, as clearly shown e.g. in Fig. 3, at the end 18 of the catheter tube the radii of the inner surface (i.e. the lumen) and the outer surface are substantially the same.

As stated above, preferably tip 26 will have a length within the range of between about 0.635 and 1.27 cm (0.25 and 0.5 inches). The tip will be of a Shore A durometer of less than 80 and be comprised of polyurethane material. Because of this, the tip will not kink or collapse and prevent the functioning of the catheter during the insertion and placement procedure. Nevertheless, the extremely soft tip with the rounded front distal end being inserted into the patient with no roughened surface at the joint between the two parts provides a much softer easier insertion and placement procedure without the risk of vessel wall perforation or damage.

The catheter body (28) is comprised of a soft polyurethane, as well, within the range of between about 80 and 90 Shore A. This, in turn, limits the amount of force transmitted to the distal end 18 of the very soft tip during the insertion and placement procedure. As discussed above, the RF bonding technique enables application of a controlled concentrated heat. Very narrow bands of both the body and soft tip materials are melted at the junction point. Molten catheter body and soft tip materials at the junction point blend at a molecular level forming an interpenetrating macromolecular structure, thus achieving a superior bonding.

It is to be understood that the length has to do with proper control versus proper softness for reducing trauma. The two limitations must be evaluated for each application, in certain instances. Nevertheless, Shore A of less than 80 polyurethane with a length as recited above, provides the desired balance between the two considerations in accordance with this invention.

## Claims

1. A vascular access catheter (10) comprising:
a substantially cylindrical tube (26, 28) having a proximal end and a distal front end (18), the tube being defined by a substantially cylindrical wall having an inner surface and an outer surface (12, 14), the wall having a thickness defined by the radial distance between the inner surface and the outer surface; said tube comprising a body (28) and a tip (26) having its front end edge rounded from the outer surface (14) to the front end (18) and said tip (26) being comprised of polyurethane having a durometer less than that of the body (28), characterised in that the outer surface (14) of the tip (26) gradually tapers to the front end (18), said body (28) is comprised of polyurethane of durometer in a range between about 80 shore A and 90 shore A and the radius of the inner surface remains constant.

2. The catheter of claim 1 wherein at the front end (18) the radii of the inner surface and the outer surface are substantially the same.

3. The catheter of claim 1, wherein the tip (26) is substantially frusto-spherical, having a frustum coextensive with the diameter of the inner surface.

4. The catheter of Claim 1, wherein said tip (26) has a length within the range of between about 0.635 and 1.27 cm (about 0.25 and 0.50 inches).

## Patentansprüche

1. Gefäßzugangskatheter (10) mit:
einem im wesentlichen zylindrischen Schlauch (26, 28) mit einem proximalen Ende und einem distalen vorderen Ende (18), wobei der Schlauch durch eine im wesentlichen zylindrische Wand mit einer Innenfläche und einer Außenfläche (12, 14) definiert ist, und wobei die Wand eine Dicke hat, die durch den radialen Abstand zwischen der Innenfläche und der Außenfläche festgelegt ist; wobei der Schlauch einen Körper (28) und eine Spitze (26) mit von der Außenfläche (14) zum vorderen Ende (18) abgerundetem vorderem Endrand hat und die Spitze (26) aus Polyurethan mit einem geringeren Härtegrad als jener des Körpers (28) besteht, dadurch gekennzeichnet, daß sich die Außenfläche (14) der Spitze (26) zum vorderen Ende (18) hin allmählich verjüngt, daß der Körper (28) aus Polyurethan mit einem Härtegrad im Bereich zwischen etwa 80 Shore A und 90 Shore A besteht, und daß der Radius der Innenfläche konstant bleibt.

2. Katheter nach Anspruch 1, wobei am vorderen Ende (18) die Radien der Innenfläche und der Außenfläche im wesentlichen gleich sind.

3. Katheter nach Anspruch 1, wobei die Spitze (26) im wesentlichen Kugelabschnitt-förmig ist, mit einem Abschnitt-Teil, der sich gleich dem Durchmesser der Innenfläche erstreckt.

4. Katheter nach Anspruch 1, wobei die Spitze (26) eine Länge im Bereich von zwischen etwa 0,635 und 1,27 cm (etwa 0,25 und 0,50 Zoll) hat.

## Revendications

1. Cathéter d'accès vasculaire (10) qui comprend :
un tube sensiblement cylindrique (26, 28) possédant une extrémité proximale et une extrémité distale avant (18), ledit tube étant défini par une paroi sensiblement cylindrique avec une surface intérieure et une surface extérieure (12, 14), la paroi ayant une épaisseur définie par la distance radiale entre ses surfaces intérieure et extérieure ; lequel tube se compose d'un corps (28) et d'un embout (26) dont le bord de l'extrémité avant est arrondi de la surface extérieure (14) à l'extrémité avant (18), ledit embout (26) étant d'un polyuréthanne de durométrie inférieure à celle du corps (28), caractérisé en ce que la surface extérieure (14) de l'embout (26) s'effile progressivement vers l'extrémité avant (18), ledit corps (28) est en polyuréthanne d'une durométrie dans la plage de 80 Shore A à 90 Shore A environ, et le rayon de la surface intérieure demeure constant.

2. Cathéter selon la revendication 1, dans lequel à l'extrémité avant (18), le rayon de la surface intérieure et de la surface extérieure est sensiblement le même.

3. Cathéter selon la revendication 1, dans lequel l'embout (26) est sensiblement tronco-cylindrique, ayant un tronc coextensif avec le diamètre de la surface intérieure.

4. Cathéter selon la revendication 1, dans lequel la longueur dudit embout (26) se situe dans la plage comprise entre 0,635 et 1,27 cm environ (entre 0,25 et 0,50 pouce environ).
